# EUROPEAN PATENT APPLICATION

(11) **EP 2 518 788 A1**
(43) Date of publication of application: **31.10.2012**
(21) Application number: 10843225.3
(22) Date of filing: 27.12.2010
(51) Int. Cl.: H01L 51/50, C07D 471/06, C07D 471/16, C07D 493/06, C08K 5/3417, C08K 5/3432, C08K 5/3447, C08L 65/00, C09K 11/06

(54) **ORGANIC ELECTROLUMINESCENT ELEMENT AND LIGHT-EMITTING POLYMER COMPOSITION**

(30) Priority: 04.02.2010 JP 2010022916; 13.01.2010 JP 2010004660
(71) Applicant: Sumitomo Chemical Company, Limited, Tokyo 104-8260 (JP)
(72) Inventor: UETANI, Yasunori, Tsukuba-shi Ibaraki 305-0045 (JP)
(74) Representative: Duckworth, Timothy John
(86) International application number: PCT/JP2010/073891
(87) International publication number: WO 2011/086873

(57) **Abstract**

An organic EL device comprising a pair of electrodes and a light emitting layer disposed between the electrodes, wherein the above-described light emitting layer is a light emitting layer comprising at least one compound selected from the group consisting of the following compounds (I) to (IV) and derivatives thereof, and a first light emitting polymer, for a light emitting layer comprising a second light emitting polymer having as a repeating unit a divalent residue of at least one compound selected from the group consisting of the above-described compounds (I) to (IV) and derivatives thereof:
(I) perylene tetracarboxylic diimide or naphthalene tetracarboxylic diimide
(II) perylene tetracarboxylic dianhydride or naphthalene tetracarboxylic dianhydride
(III) perylene tetracarboxylic bisimidazole or naphthalene tetracarboxylic bisimidazole
(IV) perylene tetracarboxylic bispyrimidine or naphthalene tetracarboxylic bispyrimidine.

## Description

### Technical Field

The present invention relates to an organic electroluminescent device (hereinafter, referred to as organic EL device in some cases), a light emitting polymer composition used for this organic EL device, and a light emitting apparatus.

### Background Art

An organic EL device has a pair of electrodes consisting of an anode and a cathode, and a light emitting layer disposed between the electrodes. When voltage is applied to the organic EL device, holes are injected from the anode and electrons are injected from the cathode into the device, and these holes and electrons are connected to cause light emission of this device.

In the light emitting layer of the organic EL device, organic substances are used as a light emitter. If organic substances used in the light emitting layer are roughly classified into light emitting polymers and light emitting small molecules, then, light emitting polymers are light emitting materials suitable for a wet method having a simple film formation step more than a dry method since light emitting polymers are dissolved relatively easily in a solvent as compared with light emitting small molecules. Therefore, there are various light emitting polymers suggested recently (for example, Advanced Materials, Vol. 12, p. 1737-1750 (2000)).

### Disclosure of the Invention

The organic EL device used as a light emitting device in various light emitting apparatuses is required to have further improved device life. The present invention has an object of providing an organic EL device having long device life, and a light emitting polymer composition used in this organic EL device.

The present invention provides the following organic electroluminescent devices, light emitting polymer compositions and the like.
<1> An organic electroluminescent device comprising a pair of electrodes and a light emitting layer disposed between the electrodes,
   wherein the above-described light emitting layer is
   a light emitting layer comprising at least one compound selected from the group consisting of the following compounds (I) to (IV) and derivatives thereof, and a first light emitting polymer , or
   a light emitting layer comprising a second light emitting polymer having as a repeating unit a divalent residue of at least one compound selected from the group consisting of the above-described compounds (I) to (IV) and derivatives thereof:
   (I) perylene tetracarboxylic diimide or naphthalene tetracarboxylic diimide
   (II) perylene tetracarboxylic dianhydride or naphthalene tetracarboxylic dianhydride
   (III) perylene tetracarboxylic bisimidazole or naphthalene tetracarboxylic bisimidazole
   (IV) perylene tetracarboxylic bispyrimidine or naphthalene tetracarboxylic bispyrimidine.
<2> The organic electroluminescent device according to <1>, wherein the derivatives of the above-described compounds (I) to (IV) are polymer compounds.
<3> The organic electroluminescent device according to <1> or <2>, wherein the above-described first and second light emitting polymers are conjugated polymers.
<4> The organic electroluminescent device according to any one of <1> to <3>, wherein the above-described first and second light emitting polymers are light emitting polymers having as a repeating unit at least one diyl group selected from the group consisting of an unsubstituted or substituted fluorenediyl group and an unsubstituted or substituted benzofluorenediyl group.
<5> The organic electroluminescent device according to any one of <1> to <4>, wherein the content of at least one compound selected from the group consisting of the above-described compounds (I) to (IV) and derivatives thereof in the above-described light emitting layer is 0.001 to 5 parts by weight when the content of the above-described light emitting polymer is 100 parts by weight.
<6> The organic electroluminescent device according to any one of <1> to <5>, wherein the content of at least one compound selected from the group consisting of the above-described compounds (I) to (IV) and derivatives thereof in the above-described light emitting layer is 0.01 to 1 part by weight when the content of the above-described light emitting polymer is 100 parts by weight.
<7> A light emitting apparatus comprising the organic electroluminescent device according to any one of <1> to <6>.
<8> A light emitting polymer composition comprising at least one compound selected from the group consisting of the following compounds (I) to (IV) and derivatives thereof, and a first light emitting polymer, or comprising
   a second light emitting polymer having as a repeating unit a divalent residue of at least one compound selected from the group consisting of the above-described compounds (I) to (IV) and derivatives thereof:
   (I) perylene tetracarboxylic diimide or naphthalene tetracarboxylic diimide
   (II) perylene tetracarboxylic dianhydride or naphthalene tetracarboxylic dianhydride
   (III) perylene tetracarboxylic bisimidazole or naphthalene tetracarboxylic bisimidazole
   (IV) perylene naphthalene tetracarboxylic bispyrimidine or naphthalene tetracarboxylic bispyrimidine.

### Modes for Carrying Out the Invention

The organic EL device of the present invention has a pair of electrodes and a light emitting layer disposed between the electrodes, and the above-described light emitting layer comprises at least one compound selected from the group consisting of the following compounds (I) to (IV) and derivatives thereof, and a first light emitting polymer:
(I) perylene tetracarboxylic diimide or naphthalene tetracarboxylic diimide
(II) perylene tetracarboxylic dianhydride or naphthalene tetracarboxylic dianhydride
(III) perylene tetracarboxylic bisimidazole or naphthalene tetracarboxylic bisimidazole
(IV) perylene tetracarboxylic bispyrimidine or naphthalene tetracarboxylic bispyrimidine.

The light emitting layer is not limited to a case comprising two compounds consisting of the above-described compound and a first light emitting polymer, may also be a light emitting layer comprising a second light emitting polymer having as a repeating unit a divalent residue of at least one compound selected from the group consisting of the above-described compounds (I) to (IV) and derivatives thereof. Further, the light emitting layer may also contain the second light emitting polymer, and a first light emitting polymer not having a divalent residue of at least one compound selected from the group consisting of the above-described compounds (I) to (IV) and derivatives thereof, in addition to this second light emitting polymer. Hereinafter, when the first and second light emitting polymers are generically named, these are called simply light emitting polymers in some cases without explicitly using "first" or "second". The light emitting polymer used in the present invention has a polystyrene-equivalent number-average molecular weight of preferably about 10³ to 10⁸, more preferably about 10³ to 10⁶, from the standpoint of film formability and solubility in a solvent. The above-described conjugated polymer has a polystyrene-equivalent weight-average molecular weight of preferably 10³ to 10⁸, more preferably 10³ to 10⁶.

The light emitting layer may contain only one compound among the above-described plural compounds, or may contain plural compounds. The derivatives of the above-described compounds (I) to (IV) include compounds obtained by linkage of a given substituent such as a heterocyclic group or an arylalkyl group, or an alkyl group, an aryl group, an alkoxy group and the like explained in the column of the formulae (1) and (2) described below to a given atom such as a carbon atom, a nitrogen atom and the like of the compounds (I) to (IV).

The heterocyclic group has a carbon atom number of usually about 4 to 60. Of heterocyclic groups, aromatic heterocyclic groups are preferable.

Specific examples of the heterocyclic group include a thienyl group, C₁ to C₁₂ alkylthienyl groups, a pyrrolyl group, a furyl group, a pyridyl group, C₁ to C₁₂ alkylpyridyl groups, a thiazolyl group, C₁ to C₁₂ alkylthiazolyl groups, a quinolyl group and the like.

The arylalkyl group has a carbon atom number of usually about 7 to 60.

Specific examples of the arylalkyl group include phenyl-C₁ to C₁₂ alkyl groups such as a phenylmethyl group, a phenylethyl group, a phenylbutyl group, a phenylpentyl group, a phenylhexyl group, a phenylheptyl group, a phenyloctyl group and the like, C₁ to C₁₂ alkoxyphenyl-C₁ to C₁₂ alkyl groups, C₁ to C₁₂ alkylphenyl-C₁ to C₁₂ alkyl groups, 1-naphthyl-C₁ to C₁₂ alkyl groups, 2-naphthyl-C₁ to C₁₂ alkyl groups and the like.

Specific examples of the perylene tetracarboxylic diimide or naphthalene tetracarboxylic diimide and derivatives thereof contained in the light emitting layer include the following compounds, compounds obtained by substituting at least one hydrogen on an aromatic ring of the following compounds by a substituent, and the like. The substituent includes an alkyl group, an alkoxy group, an aryl group, a heterocyclic group and an arylalkyl group. A hydrogen atom contained in these groups may be substituted by a fluorine atom. The definitions, specific examples and the like of the alkyl group, the alkoxy group and the aryl group are the same as the definitions, specific examples and the like of these groups in the formulae (1) and (2) described later. The definitions, specific examples and the like of the heterocyclic group and the arylalkyl group are the same as described above.

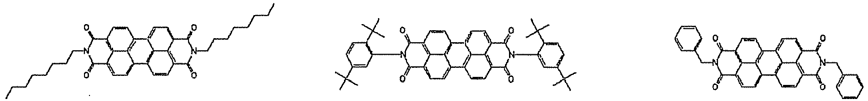

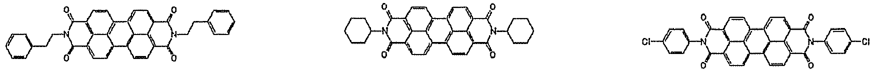

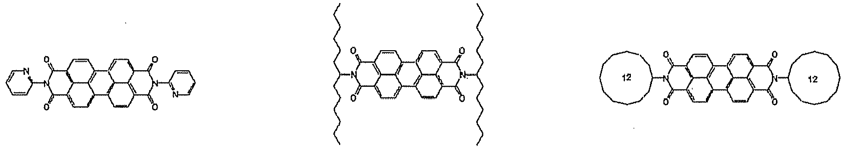

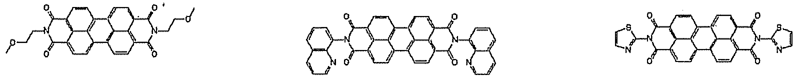

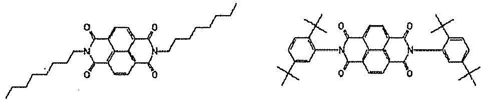

Specific examples of the perylene tetracarboxylic dianhydride or naphthalene tetracarboxylic dianhydride and derivatives thereof contained in the light emitting layer include the following compounds, compounds obtained by substituting at least one hydrogen atom on an aromatic ring of the following compounds by a substituent or an atom, and the like. The substituent includes an alkyl group, an alkoxy group, an aryl group, a heterocyclic group and an arylalkyl group. A hydrogen atom contained in these groups may be substituted by a fluorine atom. The definitions, specific examples and the like of the alkyl group, the alkoxy group and the aryl group are the same as the definitions, specific examples and the like of these groups in the formulae (1) and (2) described below. The definitions, specific examples and the like of the heterocyclic group and the arylalkyl group are the same as described above.

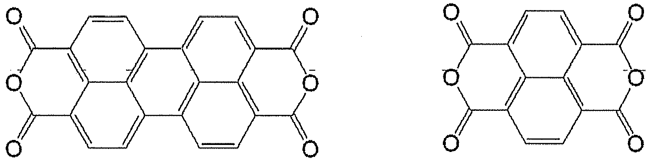

Specific structures of the perylene tetracarboxylic bisimidazole or naphthalene tetracarboxylic bisimidazole and derivatives thereof contained in the light emitting layer include the following compounds, compounds obtained by substituting at least one hydrogen atom on an aromatic ring of the following compounds by a substituent or an atom, and the like. The substituent includes an alkyl group, an alkoxy group, an aryl group, a heterocyclic group and an arylalkyl group. A hydrogen atom contained in these groups may be substituted by a fluorine atom. The definitions, specific examples and the like of the alkyl group, the alkoxy group and the aryl group are the same as the definitions, specific examples and the like of these groups in the formulae (1) and (2) described later. The definitions, specific examples and the like of the heterocyclic group and the arylalkyl group are the same as described above.

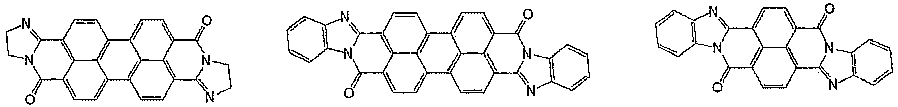

Specific structures of the perylene tetracarboxylic bispyrimidine or naphthalene tetracarboxylic bispyrimidine and derivatives thereof contained in the light emitting layer include the following compounds, compounds obtained by substituting at least one hydrogen atom on an aromatic ring of the following compounds by a substituent or an atom, and the like. The substituent includes an alkyl group, an alkoxy group or an aryl group, a heterocyclic group and an arylalkyl group. A hydrogen atom contained in these groups may be substituted by a fluorine atom. The definitions, specific examples and the like of the alkyl group, the alkoxy group and the aryl group are the same as the definitions, specific examples and the like of these groups in the formulae (1) and (2) described later. The definitions, specific examples and the like of the heterocyclic group and the arylalkyl group are the same as described above.

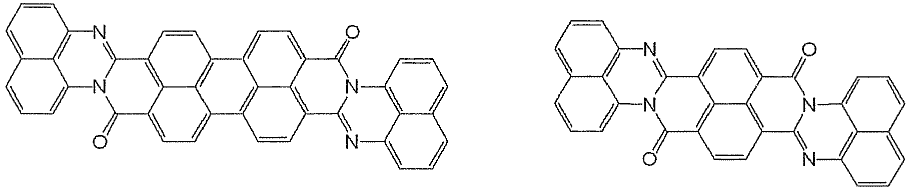

The derivative of the above-described compounds (I) to (IV) is not limited to a low molecular weight compound and may be a polymer compound. The polymer compound includes polymer compounds having as a repeating unit at least one of divalent residues of the above-described compounds (I) to (IV) and derivatives thereof. The divalent residue of the above-described compounds (I) to (IV) and derivatives thereof means a divalent group obtained by removing from the compounds (I) to (IV) and derivatives thereof two hydrogen atoms linked to a carbon atom or a nitrogen atom constituting these compounds. Specific structures of such repeating units, that is, the divalent residues of the above-described compounds (I) to (IV) and derivatives thereof include the following residues, residues obtained by substituting at least one hydrogen atom on an aromatic ring of the following residues by a substituent or an atom, and the like. The substituent includes an alkyl group, an alkoxy group or an aryl group. A hydrogen atom contained in these groups may be substituted by a fluorine atom. The definitions, specific examples and the like of the alkyl group, the alkoxy group and the aryl group are the same as the definitions, specific examples and the like of these groups in the formulae (1) and (2) described later. The definitions, specific examples and the like of the heterocyclic group and the arylalkyl group are the same as described above.

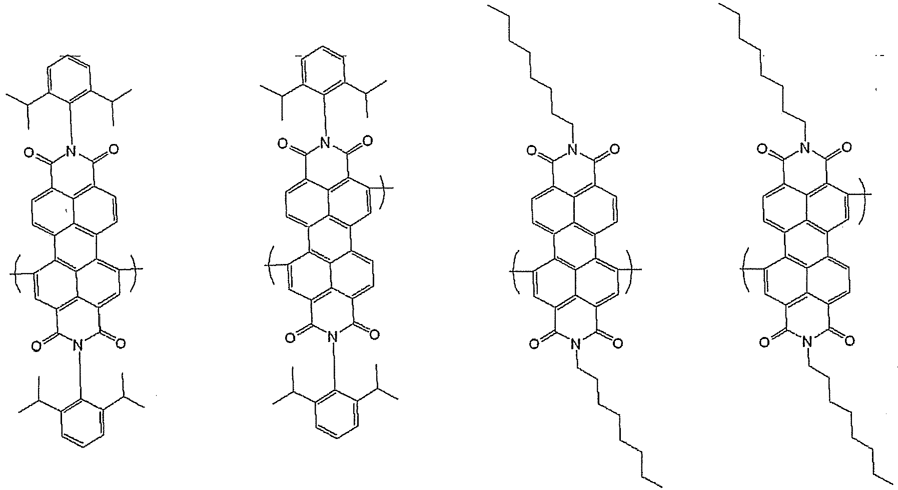

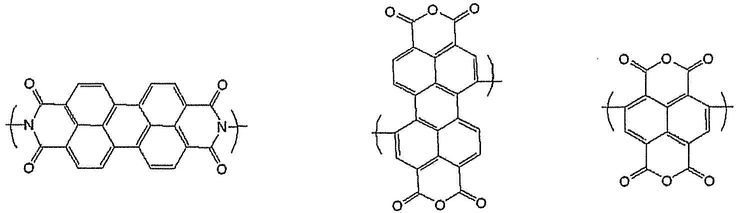

In each structural formula, straight lines crossing a parenthesis mark, that is, straight lines crossing a parenthesis mark "(" or a parenthesis mark ")" represent connecting bonds, respectively.

Next, the light emitting polymer used in the present invention will be explained. The light emitting polymer used in the present invention may be a homopolymer or a copolymer. The light emitting polymer used in the present invention may be a conjugated polymer or a non-conjugated polymer, and conjugated polymers are preferable.

It is preferable that the light emitting polymer used in the present invention has as a repeating unit at least one diyl group selected from the group consisting of an unsubstituted or substituted fluorenediyl group and an unsubstituted or substituted benzofluorenediyl group.

The unsubstituted or substituted fluorenediyl group includes, for example, diyl groups represented by the following formula (1): 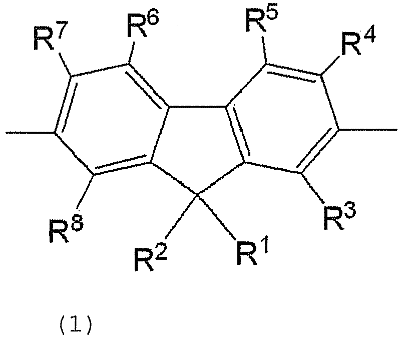 (in the formula (1), R¹, R², R³, R⁴, R⁵, R⁶, R⁷ and R⁸ represent each independently a hydrogen atom, an alkyl group, an alkoxy group or an aryl group. A hydrogen atom contained in these groups may be substituted by a fluorine atom.).

The unsubstituted or substituted benzofluorenediyl group includes diyl groups represented by the following formula (2): 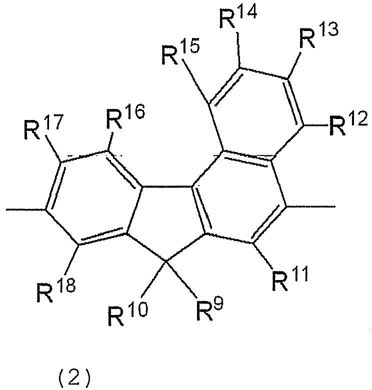 (in the formula (2), R⁹, R¹⁰, R¹¹, R¹², R¹³, R¹⁴, R¹⁵, R¹⁶, R¹⁷ and R¹⁸ represent each independently a hydrogen atom, an alkyl group, an alkoxy group or an aryl group. A hydrogen atom contained in these groups may be substituted by a fluorine atom.).

The alkyl group represented by R¹ to R¹⁸ in the formulae (1) and (2) may be linear or branched, and may also be a cycloalkyl group. The alkyl group has a carbon atom number of usually about 1 to 20.

Specific examples of the alkyl group include a methyl group, an ethyl group, a n-propyl group, an i-propyl group, a n-butyl group, an i-butyl group, a t-butyl group, a s-butyl group, a 3-methylbutyl group, a n-pentyl group, a n-hexyl group, a 2-ethylhexyl group, a n-heptyl group, a n-octyl group, a n-nonyl group, a n-decyl group, a 3,7-dimethyloctyl group, a n-lauryl group and the like. A hydrogen atom in the above-described alkyl group may be substituted by a fluorine atom. Examples of the alkyl group substituted by a fluorine atom include a trifluoromethyl group, a pentafluoroethyl group, a perfluorobutyl group, a perfluorohexyl group, a perfluorooctyl group and the like.

The alkoxy group represented by R¹ to R¹⁸ in the formulae (1) and (2) may be linear or branched, and may also be a cycloalkyloxy group. The alkoxy group has a carbon atom number of usually about 1 to 20. Specific examples of the alkoxy group include a methoxy group, an ethoxy group, a n-propyloxy group, an i-propyloxy group, a n-butoxy group, an i-butoxy group, a s-butoxy group, a t-butoxy group, a n-pentyloxy group, a n-hexyloxy group, a cyclohexyloxy group, a n-heptyloxy group, a n-octyloxy group, a 2-ethylhexyloxy group, a n-nonyloxy group, a n-decyloxy group, a 3,7-dimethyloctyloxy group, a n-lauryloxy group and the like. A hydrogen atom in the above-described alkoxy group may be substituted by a fluorine atom. Examples of the alkoxy group substituted by a fluorine atom include a trifluoromethoxy group, a pentafluoroethoxy group, a perfluorobutoxy group, a perfluorohexyl group, a perfluorooctyl group and the like.

The aryl group represented by R¹ to R¹⁸ in the formulae (1) and (2) is an atomic group obtained by removing one hydrogen atom from an aromatic hydrocarbon, and includes those having a condensed ring, and those having two or more independent benzene rings or condensed rings linked directly or via a group such as vinylene or the like. The aryl group has a carbon atom number of usually about 6 to 60, preferably 6 to 48. The above-described aryl group may have a substituent. This substituent includes linear or branched alkyl groups having 1 to 20 carbon atoms or cycloalkyl groups having 1 to 20 carbon atoms, alkoxy groups containing a linear or branched alkyl group having 1 to 20 carbon atoms or a cycloalkyl group having 1 to 20 carbon atoms in its structure, and groups represented by the following formula (3). Specific examples of the aryl group include a phenyl group, C₁ to C₁₂ alkoxyphenyl groups (C₁ to C₁₂ indicates that the carbon atom number is 1 to 12. The same shall apply hereinafter.), C₁ to C₁₂ alkylphenyl groups, a 1-naphthyl group, a 2-naphthyl group, a 1-anthracenyl group, a 2-anthracenyl group, a 9-anthracenyl group, a pentafluorophenyl group and the like, and preferable are C₁ to C₁₂ alkoxyphenyl groups and C₁ to C₁₂ alkylphenyl groups. The C₁ to C₁₂ alkoxyphenyl groups include specifically a methoxyphenyl group, an ethoxyphenyl group, a n-propyloxyphenyl group, an isopropyloxyphenyl group, a n-butoxyphenyl group, an isobutoxyphenyl group, a s-butoxyphenyl group, a t-butoxyphenyl group, a n-pentyloxyphenyl group, a n-hexyloxyphenyl group, a cyclohexyloxyphenyl group, a n-heptyloxyphenyl group, a n-octyloxyphenyl group, a 2-ethylhexyloxyphenyl group, a n-nonyloxyphenyl group, a n-decyloxyphenyl group, a 3,7-dimethyloctyloxyphenyl group, a n-lauryloxyphenyl group and the like. The C₁ to C₁₂ alkylphenyl groups include specifically a methylphenyl group, an ethylphenyl group, a dimethylphenyl group, a n-propylphenyl group, a mesityl group, a methylethylphenyl group, an isopropylphenyl group, a n-butylphenyl group, an isobutylphenyl group, a s-butylphenyl group, a t-butylphenyl group, a n-pentylphenyl group, an isoamylphenyl group, a hexylphenyl group, a n-heptylphenyl group, a n-octylphenyl group, a n-nonylphenyl group, a n-decylphenyl group, a n-dodecylphenyl group and the like. A hydrogen atom in the above-described aryl group may be substituted by a fluorine atom.

—O—(CH₂)_{g}—O—(CH₂)ₕ—CH₃ (3)

(in the formula (3), g represents an integer of 1 to 6 and h represents an integer of 0 to 5.).

R¹, R², R⁹ and R¹⁰ in the formulae (1) and (2) represent preferably an alkyl group, an alkoxy group or an aryl group, more preferably an alkyl group or an aryl group, from the standpoint of solubility in an organic solvent.

Examples of the diyl group represented by the formula (1) include the following diyl groups.

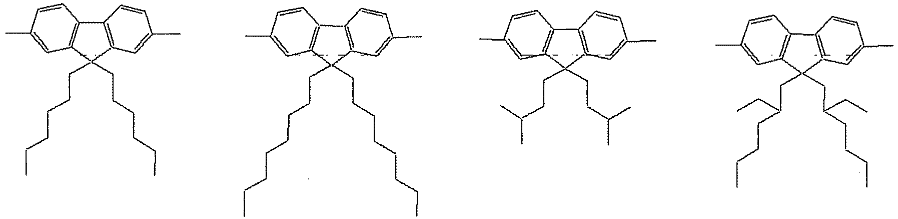

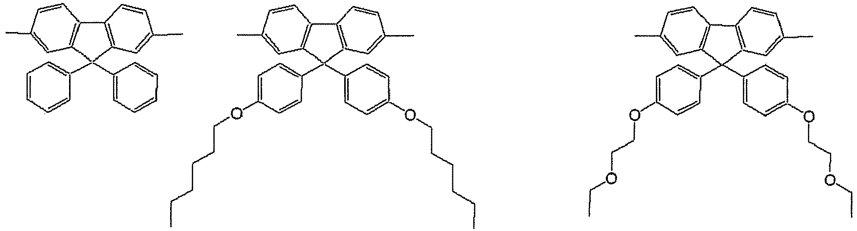

Examples of the diyl group represented by the formula (2) include the following diyl groups.

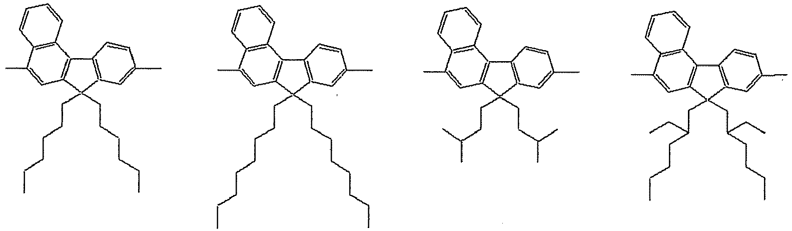

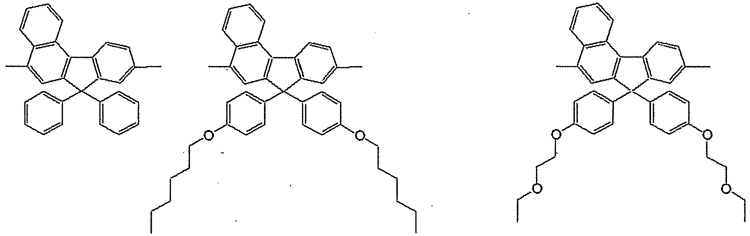

The conjugated polymer means (1) a polymer composed substantially of a structure in which double bonds and single bonds are arranged alternately, (2) a polymer composed substantially of a structure in which double bonds and single bonds are arranged via a nitrogen atom, (3) a polymer composed substantially of a structure in which double bonds and single bonds are arranged alternately and a structure in which double bonds and single bonds are arranged via a nitrogen atom, or the like, and preferable conjugated polymers having at least as a repeating unit at least one diyl group selected from the group consisting of an unsubstituted or substituted fluorenediyl group and an unsubstituted or substituted benzofluorenediyl group and in which the repeating units are mutually linked directly or via a linkage group. This conjugated polymer may have as a repeating unit one or more selected from the group consisting of, for example, a dibenzofurandiyl group, an unsubstituted or substituted dibenzothiophenediyl group, an unsubstituted or substituted carbazolediyl group, an unsubstituted or substituted thiophenediyl group, an unsubstituted or substituted furandiyl group, an unsubstituted or substituted pyrrolediyl group, an unsubstituted or substituted benzothiadiazolediyl group, an unsubstituted or substituted phenylenevinylenediyl group, an unsubstituted or substituted thienylenevinylenediyl group and an unsubstituted or substituted triphenylaminediyl group, in addition to at least one group selected from the group consisting of an unsubstituted or substituted fluorenediyl group and an unsubstituted or substituted benzofluorenediyl group, and may also be a polymer in which the above-described unsubstituted or substituted fluorenediyl group and unsubstituted or substituted benzofluorenediyl group, and additionally, these repeating units are mutually linked directly or via a linkage group.

When, in the conjugated polymer, repeating units are mutually linked via a linkage group, examples of the linkage group include phenylene, biphenylene, naphthalenediyl, anthracenediyl and the like.

The first light emitting polymer among the first and second light emitting polymers does not have a divalent residue of at least one compound selected from the group consisting of the above-described compounds (I) to (IV) and derivatives thereof, while the second light emitting polymer has as a repeating unit a divalent residue of at least one compound selected from the group consisting of the compounds (I) to (IV) and derivatives thereof. Such a divalent residue includes the above-described divalent residues. The second light emitting polymer may have as a repeating unit a divalent residue of at least one compound selected from the group consisting of the compounds (I) to (IV) and derivatives thereof and further may have as a repeating unit other divalent groups, and for example, may have as a repeating unit at least one diyl group selected from the group consisting of the above-described unsubstituted or substituted fluorenediyl group and unsubstituted or substituted benzofluorenediyl group.

The conjugated polymer has a polystyrene-equivalent number-average molecular weight preferably of about 10³ to 10⁸, more preferably of about 10³ to 10⁶, from the standpoint of film formability and solubility in a solvent. The above-described conjugated polymer has a polystyrene-equivalent weight-average molecular weight of preferably 10³ to 10⁸, more preferably 10³ to 10⁶.

When the conjugated polymer contains an unsubstituted or substituted fluorenediyl group and/or an unsubstituted or substituted benzofluorenediyl group, the proportion, with respect to the conjugated polymer, of the total molecular weight of the unsubstituted or substituted fluorenediyl group and the unsubstituted or substituted benzofluorenediyl group constituting this conjugated polymer is usually 0.3 or more, preferably 0.5 or more when the molecular weight of the conjugated polymer is 1.

The proportion, with respect to the second light emitting polymer, of the total molecular weight of a divalent residue of at least one compound selected from the group consisting of compounds (I) to (IV) and derivatives thereof constituting the second light emitting polymer is usually 0.0001 to 1.0, more preferably 0.0001 to 0.5, further preferably 0.001 to 0.2 when the molecular weight of the second light emitting polymer is 1.

The light emitting polymer used in the present invention can be produced by known polymerization methods.

For example, the light emitting polymer as a conjugated polymer can be synthesized by synthesizing monomers having a functional group suitable for the polymerization reaction to be used, then, if necessary, dissolving the monomers in an organic solvent, and polymerizing the monomers by using a polymerization method such as, for example, known aryl coupling and the like using an alkali and a suitable catalyst and a ligand

The polymerization method by aryl coupling is not particularly restricted. The polymerization method includes a method of polymerizing a monomer having for example a boric group or a borate group as a functional group with a monomer having a halogen atom such as a bromine atom, an iodine atom, a chlorine atom and the like or a sulfonate group such as a trifluoromethanesulfonate group, a p-toluenesulfonate group and the like as a functional group, the functional groups being suitable for the polymerization reaction, in the presence of an inorganic base such as sodium carbonate, potassium carbonate, cesium carbonate, tripotassium phosphate, potassium fluoride and the like or an organic base such as tetrabutylammonium fluoride, tetrabutylammonium chloride, tetrabutylammonium bromide, tetraethylammonium hydroxide and the like, using a catalyst composed of a Pd or Ni complex such as palladium[tetrakis(triphenylphosphine)], [tris(dibenzylideneacetone)]dipalladium, palladium acetate, bis(triphenylphosphine)palladium dichloride, bis(cyclooctadiene)nickel and the like and if necessary, further a ligand such as triphenylphosphine, tri(2-methylphenyl)phosphine, tri(2-methoxyphenyl)phosphine, diphenylphosphinopropane, tri(cyclohexyl)phosphine, tri(tert-butyl)phosphine and the like, according to the Suzuki coupling reaction; a method of mutually reacting monomers having a halogen atom or a sulfonate group such as a trifluoromethanesulfonate group and the like using a catalyst composed of a nickel zero-valent complex such as bis(cyclooctadiene)nickel and the like and a ligand such as bipyridyl and the like or using a catalyst composed of a Ni complex such as [bis(diphenylphosphino)ethane]nickel dichloride, [bis(diphenylphosphino)propane]nickel dichloride and the like and if necessary, further a ligand such as triphenylphosphine, diphenylphosphinopropane, tri(cyclohexyl)phosphine, tri(tert-butyl)phosphine and the like and a reducing agent such as zinc, magnesium and the like, if necessary under dehydration conditions, according to the Yamamoto coupling reaction; a method of reacting a compound having a magnesium halide group with a compound having a halogen atom using a Ni catalyst such as [bis(diphenylphosphino)ethane]nickel dichloride, [bis(diphenylphosphino)propane]nickel dichloride and the like, under dehydration conditions, by aryl coupling according to the Kumada-Tamao coupling reaction; a method of polymerization with an oxidizer such as FeCl₃ and the like utilizing a hydrogen atom as a functional group; a method of electro-chemical oxidation polymerization; and the like.

The reaction solvent is selected in view of the polymerization reaction to be used, the solubility of monomers and polymers, and the like. Specific examples thereof include organic solvents such as tetrahydrofuran, toluene, 1,4-dioxane, dimethoxyethane, N,N-dimethylacetamide, N,N-dimethylformamide, mixed solvents composed of two or more of them, and the like, or two-phase systems composed of them with water.

In the Suzuki coupling reaction, preferable are organic solvents such as tetrahydrofuran, toluene, 1,4-dioxane, dimethoxyethane, N,N-dimethylacetamide, N,N-dimethylformamide, mixed solvents composed of two or more of them, and the like, or two-phase systems composed of them with water. It is preferable that the reaction solvent is subjected to a deoxygenation treatment, in general, for suppressing side reactions.

In the Yamamoto coupling reaction, preferable are organic solvents such as tetrahydrofuran, toluene, 1,4-dioxane, dimethoxyethane, N,N-dimethylacetamide, N,N-dimethylformamide, mixed solvents composed of two or more of them, and the like. It is preferable that the reaction solvent is subjected to a deoxygenation treatment, in general, for suppressing side reactions.

Among aryl coupling reactions, the Suzuki coupling reaction and the Yamamoto coupling reaction are preferable, and the Suzuki coupling reaction and the Yamamoto coupling reaction using a nickel zero-valent complex are more preferable, from the standpoint of reactivity. For further details of polymerization according to Suzuki coupling, known methods described in, for example, Journal of Polymer Science: Part A: Polymer Chemistry, Vol. 39, 1533-1556 (2001) can be referred. Regarding polymerization according to Yamamoto coupling, known methods described in, for example, Macromolecules 1992, 25, 1214-1223 can be referred.

The reaction temperature in these reactions is not particularly restricted providing that it is in a temperature range wherein the reaction solution remains liquid, and its lower limit is preferably -100°C, more preferably -20°C, particularly preferably 0°C from the standpoint of reactivity and its upper limit is preferably 200°C, more preferably 150°C, particularly preferably 120°C from the standpoint of the stability of the above-described conjugated polymer and the compound represented by the above-described formula (1) or (2).

Taking out of the conjugated polymer can be carried out according to a known method. For example, the above-described conjugated polymer can be obtained by adding the reaction solution to a lower alcohol such as methanol and the like to cause deposition of a precipitate and filtrating and drying the precipitate. When the purity of the resultant conjugated polymer is lower, the polymer can be purified by usual methods such as recrystallization, continuous extraction by a Soxhlet extractor, column chromatography and the like.

The light emitting polymer composition of the present invention contains at least one compound selected from the group consisting of the above-described compounds (I) to (IV) and derivatives thereof, and the above-described first light emitting polymer. The light emitting polymer composition may further contain a solvent or a dispersion medium in addition to at least one compound selected from the group consisting of the above-described compounds (I) to (IV) and derivatives thereof, and the above-described first light emitting polymer, or may also be a liquid composition containing at least one compound selected from the group consisting of the above-described compounds (I) to (IV) and derivatives thereof and the first polymer light emitter dissolved in a solvent or dispersed in a dispersion medium.

The light emitting polymer composition may contain only the above-described second light emitting polymer, or may contain the second polymer light emitting composition and other components, for example, the first light emitting polymer. Alternatively, the light emitting polymer composition may further contain a solvent or a dispersing medium in addition to the above-described second light emitting polymer.

As the above-described solvent, those which are stable and capable of uniformly dissolving or dispersing at least one compound selected from the group consisting of the above-described compounds (I) to (IV) and derivatives thereof, and the above-described first light emitting polymer can be appropriately selected from Known solvents and used. Further, as the above-described solvent, those which are stable and capable of uniformly dissolving or dispersing the second light emitting polymer can be appropriately selected from known solvents and used. Such solvents include alcohols (methanol, ethanol, isopropyl alcohol and the like), ketones (acetone, methyl ethyl ketone and the like), organic chlorines (chloroform, 1,2-dichloroethane and the like), aromatic hydrocarbons (benzene, toluene, xylene and the like), aliphatic hydrocarbons (n-hexane, cyclohexane and the like), amides (dimethylformamide and the like), sulfoxides (dimethyl sulfoxide and the like), and the like. These solvents may be used singly or in combination.

The content of at least one compound selected from the group consisting of the above-described compounds (I) to (IV) and derivatives thereof in the above-described light emitting layer is usually 0.001 to 10 parts by weight, preferably 0.001 to 5 parts by weight, further preferably 0.01 to 1 part by weight, further preferably 0.05 parts by weight to 1 part by weight with respect to 100 parts by weight of the above-described first light emitting polymer.

The content of the second light emitting polymer in the above-described light emitting layer is usually 0.001 to 100 parts by weight, preferably 0.001 to 20 parts by weight, further preferably 0.001 to 10 parts by weight, further more preferably 0.01 to 5 parts by weight with respect to 100 parts by weight of the light emitting layer.

When the light emitting polymer composition of the present invention contains a solvent, the amount of the solvent is about 90 to 99.9 with respect to 100 parts by weight of the light emitting polymer composition, and for example, the amount of the solvent in the light emitting polymer composition is usually about 1000 to 100000 parts by weight with respect to 100 parts by weight of the total amount of at least one compound selected from the group consisting of the above-described compounds (I) to (IV) and derivatives thereof, and the above-described first light emitting polymer.

The light emitting polymer composition of the present invention may contain other components, in addition to at least one compound selected from the group consisting of the above-described compounds (I) to (IV) and derivatives thereof, and the above-described first light emitting polymer, in the range not deteriorating charge transportability and charge injectability. In the light emitting polymer composition or a light emitting layer formed by using this composition, the total amount of at least one compound selected from the group consisting of the above-described compounds (I) to (IV) and derivatives thereof, and the first light emitting polymer is usually 30 parts by weight or more, preferably 50 parts by weight or more, further preferably 70 parts by weight or more with respect to 100 parts by weight of the light emitting polymer composition or the light emitting layer.

The organic EL device of the present invention has a pair of electrodes consisting of an anode and a cathode and a light emitting layer disposed between the electrodes, as described above. The light emitting layer is a light emitting layer comprising at least one compound selected from the group consisting of the above-described compounds (I) to (IV) and derivatives thereof, and a first light emitting polymer, or is a light emitting layer comprising a second light emitting polymer having as a repeating unit a divalent residue of at least one compound selected from the group consisting of the above-described compounds (I) to (IV) and derivatives thereof.

The organic EL device may have a given layer between electrodes in addition to the light emitting layer.

The layer to be disposed between a cathode and the light emitting layer includes an electron injection layer, an electron transporting layer, a hole block layer and the like. When both an electron injection layer and an electron transporting layer are disposed between a cathode and the light emitting layer, the layer in contact with a cathode is called an electron injection layer and the layer excluding this electron injection layer is called an electron transporting layer. The electron injection layer has a function of improving efficiency of injection of electrons from a cathode. The electron transporting layer has a function of improving injection of electrons from a layer in contact with the surface on the cathode side. The hole block layer has a function of blocking transportation of holes. When the electron injection layer and/or the electron transporting layer has a function of blocking transportation of holes, these layers serve as the hole block layer concurrently in some cases. Regarding the function of blocking transportation of holes by the hole block layer, for example, a device allowing only flow of hall current is fabricated and the blocking effect can be confirmed on the basis of reduction in its current value.

The layer to be disposed between an anode and the light emitting layer includes a hole injection layer, a hole transporting layer, and an electron block layer and the like. When both a hole injection layer and a hole transporting layer are disposed between an anode and the light emitting layer, the layer in contact with an anode is called a hole injection layer and the layer excluding this hole injection layer is called a hole transporting layer. The hole injection layer has a function of improving efficiency of injection of holes from an anode. The hole transporting layer has a function of improving injection of holes from a layer in contact with the surface on the anode side. The electron block layer has a function of blocking transportation of electrons. When the hole injection layer and/or the hole transporting layer has a function of blocking transportation of electrons, these layers serve as the electron block layer concurrently in some cases. Regarding the function of blocking transportation of electrons by the electron block layer, for example, a device allowing only flow of electron current is fabricated, and the blocking effect can be confirmed on the basis of reduction in its current value.

The electron injection layer and the hole injection layer are generically named a charge injection layer in some cases, and the electron transporting layer and the hole transporting layer are generically named a charge transporting layer in some cases.

Examples of possible layer constitutions of the organic EL device are shown below.
a) anode/light emitting layer/cathode
b) anode/hole injection layer/light emitting layer/cathode
c) anode/hole injection layer/light emitting layer/electron injection layer/cathode
d) anode/hole injection layer/light emitting layer/electron transporting layer/cathode
e) anode/hole injection layer/light emitting layer/electron transporting layer/electron injection layer/cathode
f) anode/hole transporting layer/light emitting layer/cathode
g) anode/hole transporting layer/light emitting layer/electron injection layer/cathode
h) anode/hole transporting layer/light emitting layer/electron transporting layer/cathode
i) anode/hole transporting layer/light emitting layer/electron transporting layer/electron injection layer/cathode
j) anode/hole injection layer/hole transporting layer/light emitting layer/cathode
k) anode/hole injection layer/hole transporting layer/light emitting layer/electron injection layer/cathode
l) anode/hole injection layer/hole transporting layer/light emitting layer/electron transporting layer/cathode
m) anode/hole injection layer/hole transporting layer/light emitting layer/electron transporting layer/electron injection layer/cathode
n) anode/light emitting layer/electron injection layer/cathode
o) anode/light emitting layer/electron transporting layer/cathode
p) anode/light emitting layer/electron transporting layer/electron injection layer/cathode
(here, the mark "/" indicates that layers sandwiching the mark "/" are adjacently laminated. The same shall apply hereinafter.).

The organic EL device may have two or more light emitting layers. For example, if a laminated body sandwiched by an anode and a cathode in any one of the above-described a) to p) is called "structural unit A", a layer constitution shown in the following q) is mentioned as the constitution of the organic EL device having two light emitting layers. Two (structural unit A) layer constitutions may be mutually the same or different.
q) anode/(structural unit A)/charge generating layer/(structural unit A)/cathode
   If "(structural unit A)/charge generating layer" is called "structural unit B", the constitution of the organic EL device having three or more light emitting layers includes a layer constitution shown in the following r).
r) anode/(structural unit B)x/(structural unit A)/cathode

The mark "x" represents an integer of 2 or more, and (structural unit B)x denotes a laminate having x structural units B laminated. A plurality of (structural unit B) layer constitutions may be the same or different.

The organic EL device can be usually fabricated by sequentially laminating the above-described layers constituting the organic EL device by a given method on a supporting substrate. For example, the organic EL device can be fabricated by laminating layers sequentially from the right side layer on a supporting substrate or by laminating layers sequentially from the left side layer on a supporting substrate, in the above-described constitutions a) to r).

### <Anode>

In the case of an organic EL device having a constitution in which a light emitted from a light emitting layer passes through an anode and exits out of a device, an electrode showing light permeability is used as an anode. As the electrode showing light permeability, use can be made of thin films made of a metal oxide, a metal sulfide, a metal and the like, and those showing high electric conductivity and high light transmission are suitably used. Specifically, used is made of thin films made of indium oxide, zinc oxide, tin oxide, indium tin oxide (Indium Tin Oxide: abbreviation ITO), indium zinc oxide (Indium Zinc Oxide: abbreviation IZO), gold, platinum, silver and copper, and the like, and of them, thin films made of ITO, IZO or tin oxide are suitably used. Anode fabrication methods include a vacuum vapor deposition method, a sputtering method, an ion plating method, a plating method and the like. As the anode, transparent electrically conductive films made of organic substances such as polyaniline or derivatives thereof, polythiophene or derivatives thereof, and the like may also be used.

The anode thickness is appropriately set in view of properties required and simplicity of the process and the like, and is for example 10 nm to 10 µm, preferably 20 nm to 1 µm, further preferably 50 nm to 500 nm.

### <Cathode>

As the cathode material, preferable are materials having small work function, causing easy injection of electrons into a light emitting layer, and having high electric conductivity. In the case of an organic EL device having a constitution in which a light is emitted from the anode side, preferable are materials showing high visible light reflectance as the cathode material, for allowing a light radiated from a light emitting layer to be reflected on the cathode toward the anode side. As the cathode, for example, alkali metals, alkaline earth metals, transition metals and group XIII metals in the periodic table and the like can be used. As the cathode material, use is made of, for example, metals such as lithium, sodium, potassium, rubidium, cesium, beryllium, magnesium, calcium, strontium, barium, aluminum, scandium, vanadium, zinc, yttrium, indium, cerium, samarium, europium, terbium, ytterbium and the like, alloys composed of two or more of the above-described metals, alloys composed of at least one of the above-described metals and at least one of gold, silver, platinum, copper, manganese, titanium, cobalt, nickel, tungsten and tin, or graphite or graphite intercalation compounds and the like. Examples of the alloys include a magnesium-silver alloy, a magnesium-indium alloy, a magnesium-aluminum alloys, an indium-silver alloy, a lithium-aluminum alloy, a lithium-magnesium alloy, a lithium-indium alloy, a calcium-aluminum alloy and the like. As the cathode, transparent electrically conductive electrodes composed of electrically conductive metal oxides and electrically conductive organic substances and the like can be used. Specifically, the electrically conductive metal oxides include indium oxide, zinc oxide, tin oxide, ITO and IZO, and the electrically conductive organic substances include polyaniline or derivatives thereof, polythiophene or derivatives thereof, and the like. The cathode may be constituted of a laminate composed of two or more layers laminated. An electron injection layer may be used as the cathode in some cases.

The cathode thickness is appropriately set in view of properties required and simplicity of the process and the like, and is for example 10 nm to 10 µm, preferably 20 nm to 1 µm, further preferably 50 nm to 500 nm.

Cathode fabrication methods include a vacuum vapor deposition method, a sputtering method, a laminate method of thermocompression bonding a metal film, and the like.

### <Light emitting layer>

It is preferable that a light emitting layer is formed by a coating method. The coating method is preferable in that the production process can be simplified and productivity is excellent. The coating method includes a casting method, a spin coat method, a bar coat method, a blade coat method, a roll coat method, a gravure printing, a screen printing, an inkjet method and the like. When a light emitting layer is formed using the above-described coating method, first, a light emitting polymer composition in solution state comprising at least one compound selected from the group consisting of the above-described compounds (I) to (IV) and derivatives thereof, and the above-described light emitting polymer is prepared as a coating solution, this coating solution is coated on a desired layer or electrode by the above-described given coating method, further, this is dried to form a light emitting layer having desired thickness.

### <Other layers>

The materials of the hole injection layer, the hole transporting layer, the electron injection layer, the electron transporting layer and the like are not particularly restricted. The hole injection layer material includes, for example, oxide such as vanadium oxide, molybdenum oxide, ruthenium oxide, aluminum oxide and the like, phenylamine compounds, starburst amine compounds, phthalocyanine compounds, amorphous carbon, polyaniline, and polythiophene derivatives. The hole transporting layer material includes polyvinylcarbazole or derivatives thereof, polysilane or derivatives thereof, polysiloxane derivatives having an aromatic amine in the side chain or the main chain, pyrazoline derivatives, arylamine derivatives, stilbene derivatives, triphenyldiamine derivatives, polyaniline or derivatives thereof, polythiophene or derivatives thereof, polyarylamine or derivatives thereof, polypyrrole or derivatives thereof, poly(p-phenylenevinylene) or derivatives thereof, or poly(2,5-thienylenevinylene) or derivatives thereof. The electron transporting layer material includes oxadiazole derivatives, anthraquinodimethane or derivatives thereof, benzoquinone or derivatives thereof, naphthoquinone or derivatives thereof, anthraquinone or derivatives thereof, tetracyanoanthraquinodimethane or derivatives thereof, fluorenone derivatives, diphenyldicyanoethylene or derivatives thereof, diphenoquinone derivatives, or metal complexes of 8-hydroxyquinoline or derivatives thereof, polyquinoline or derivatives thereof, polyquinoxaline or derivatives thereof, and polyfluorene or derivatives thereof. The electron injection layer material includes alkali metals, alkaline earth metals, alloys containing at least one of alkali metals and alkaline earth metals, oxides, halides and carbonates of alkali metals or alkaline earth metals, or mixtures of these substances, and the like. Each layer is formed by a given film formation method such as a coating method, a vacuum vapor deposition method, a sputtering method, a laminate method and the like.

The organic EL device explained above can be used suitably for curved or planar illumination apparatuses, planar light sources used for example as a light source of a scanner, and light emitting apparatuses such as displays and the like.

Displays having the organic EL device include a segment display, a dot matrix display and the like. The dot matrix display includes an active matrix display and a passive matrix display and the like. The organic EL device is used as a light emitting device constituting each picture element in an active matrix display and a passive matrix display. The organic EL device is used as a light emitting device constituting each segment or as backlight in a segment display. The organic EL device is used as backlight in a liquid crystal display.

### EXAMPLES

Examples will be shown for illustrating the present invention further in detail below, but the present invention is not limited to them.

### -Method of measuring molecular weight-

In examples, the number average molecular weight (Mn) and the weight average molecular weight (Mw) were measured gel permeation chromatography (GPC) in terms of polystyrene. Specifically, three columns of TSKgel Super HM-H (manufactured by Tosoh Corporation) were serially connected and tetrahydrofuran was allowed to flow at a flow rate 0.5 mL/min as a developing solvent and Mn and Mw were measured at 40°C according to GPC (manufactured by Tosoh Corporation, trade name: HLC-8220 GPC). As the detector, a differential refractive index detector was used.

### <Synthesis Example 1> (Synthesis of polymer compound 1)

Into a 500 ml four-necked flask were placed 1.72 g of methyltrioctylammonium chloride (trade name: Aliquat 336 manufactured by Sigma Aldrich Japan), 6.2171 g of a compound A represented by the following formula: 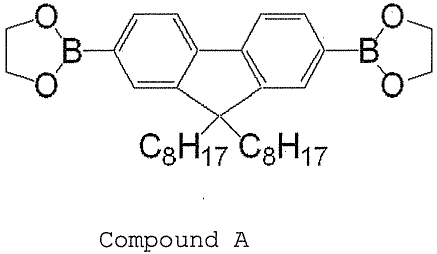 0.5085 g of a compound B represented by the following formula: 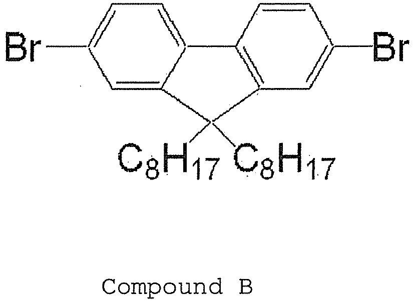 6.2225 g of a compound C represented by the following formula: 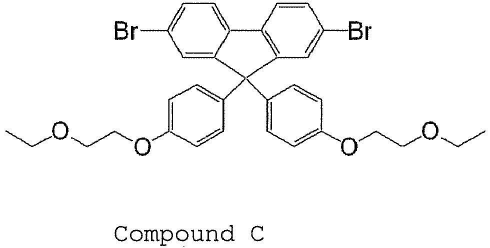 and 0.5487 g of a compound D represented by the following formula: 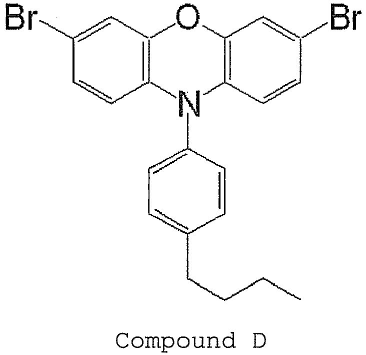 , and an atmosphere in the flask was purged with nitrogen. Toluene (100 ml) was added, and 7.6 mg of dichlorobis(triphenylphosphine)palladium(II) and 24 ml of a sodium carbonate aqueous solution were added and the mixture was stirred for 3 hours under reflux, then, 0.40 g of phenylboric acid was added and the mixture was stirred overnight. A sodium N,N-diethyldithiocarbamate aqueous solution was added, further, the mixture was stirred for 3 hours under reflux. The resultant reaction liquid was separated, and the organic phase was washed with an acetic acid aqueous solution and water, then, dropped into methanol, to find generation of a precipitate. The resultant precipitate was filtrated, dried under reduced pressure, then, dissolved in toluene, passed through a silica gel-alumina column, and washed with toluene. The resultant toluene solution was dropped into methanol, to find generation of a precipitate. The resultant precipitate was filtrated, dried under reduced pressure, then, dissolved in toluene, and dropped into methanol, to find generation of a precipitate. The resultant precipitate was filtrated, dried under reduced pressure, to obtain 7.72 g of a polymer compound 1 (conjugated polymer). The polymer compound 1 had a polystyrene-equivalent number-average molecular weight Mn of 1.2×10⁵ and a polystyrene-equivalent weight-average molecular weight Mw of 2.9×10⁵.

### <Synthesis Example 2> (Synthesis of polymer compound 2)

Into a 5 L separable flask were placed 40.18 g of methyltrioctylammonium chloride (trade name: Aliquat 336 manufactured by Sigma Aldrich Japan), 234.06 g of a compound A represented by the following formula: 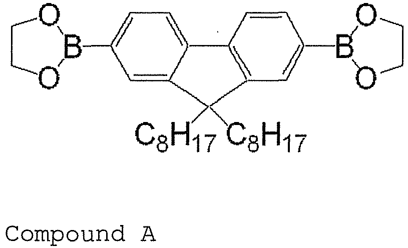 172.06 g of a compound E represented by the following formula: 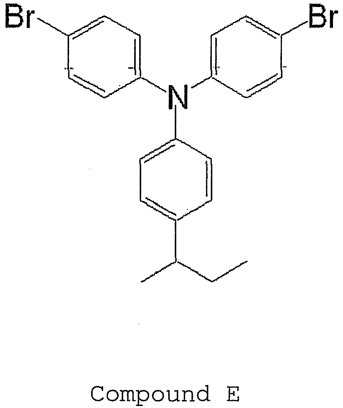 and 28.5528 g of a compound F represented by the following formula: 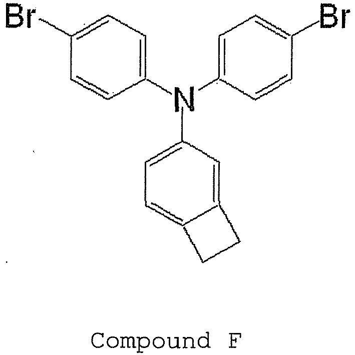 and an atmosphere in the flask was purged with nitrogen. Toluene (2620 g) through which argon had been bubbled was added, and the mixture was further bubbled for 30 minutes while stirring. Palladium acetate (99.1 mg) and tris(o-tolyl)phosphine (937.0 mg) were added, and the mixture was washed with 158 g of toluene, and heated at 95°C. A 17.5 wt% sodium carbonate aqueous solution (855 g) was dropped, then, the bath temperature was raised to 110°C, and the mixture was stirred for 9.5 hours, then, 5.39 g of phenylboric acid dissolved in 96 ml of toluene was added, and the mixture was stirred for 14 hours. Toluene (200 ml) was added, the reaction liquid was separated, and the organic phase was washed with 850 ml of a 3 wt% acetic acid aqueous solution twice, further, 850 ml of water and 19.89 g of sodium N,N-diethyldithiocarbamate were added, and the mixture was stirred for 4 hours. After liquid-separation, it was passed through a silica gel-alumina column, and washed with toluene. The resultant toluene solution was dropped into 50 L of methanol, to find generation of a precipitate. The resultant precipitate was washed with methanol. After drying under reduced pressure, it was dissolved in 11 L of toluene, and the resultant toluene solution was dropped into 50 L of methanol, to find generation of a precipitate. The resultant precipitate was filtrated, and dried under reduced pressure to obtain 278.39 g of a polymer compound 2. The polymer compound 2 had a polystyrene-equivalent number-average molecular weight Mn of 7.7×10⁴ and a polystyrene-equivalent weight-average molecular weight Mw of 3.8×10⁵.

The above-described compounds A to F can be synthesized by a method described, for example, in WO 2005/52027.

### <Synthesis of polymer compound 3>

Into a 200 mL separable flask were added 1.591 g (3.00 mmol) of 9,9-dioctylfluorene-2,7-diboric acid ethylene glycol ester, 1.612 g (2.94 mmol) of 9,9-dioctyl-2,7-dibromofluorene, 0.052 g (0.06 mmol) of N,N'-bis(2,6-diisopropylphenyl)-dibromoperylene-3,4:9,10-tetracarboxylic bisimide (an isomer mixture of a 1,6-dibromo body and a 1,7-dibromo body), 0.39 g of methyltrioctylammonium chloride (trade name: Aliquat 336, manufactured by Sigma Aldrich Japan) and 30 mL of toluene. Under a nitrogen atmosphere, 3.2 mg of bistriphenylphosphinepalladium dichloride was added and the mixture was heated at 85°C. This solution was heated at 105°C while dropping 8.2 mL of a 17.5 wt% sodium carbonate aqueous solution into this, then, the mixture was stirred for 2.5 hours. Next, 0.366 g of phenylboric acid and 30 mL of toluene were added and the mixture was stirred at 105°C overnight.

After removal of the aqueous layer, 1.67 g of sodium N,N-diethyldithiocarbamate trihydrate and 33 mL of ion exchanged water were added and the mixture was stirred at 85°C for 2 hours. The organic layer was separated from the aqueous layer, then, the organic layer was washed with ion exchanged water (twice), a 3 wt% acetic acid aqueous solution (twice) and ion exchanged water (twice) in this order.

The organic layer was dropped into methanol to cause precipitation of a polymer, and the precipitate was filtrated, then, dried to obtain a solid.

This solid was dissolved in toluene, and the solution was passed through a silica gel/alumina column though which toluene had been passed previously, and the passed eluate was dropped into methanol to cause precipitation of a polymer, and the precipitate was filtrated, then, dried to obtain 1.78 g of a polymer compound (hereinafter, referred to as polymer compound 3). The polystyrene-equivalent number-average molecular weight and weight average molecular weight were Mn=9.7×10⁴ and Mw=2.5×10⁵, respectively.

The polymer compound 3 is a polymer having the following repeating units at the following molar ratio (99:1) (the theoretical value calculated from raw materials).

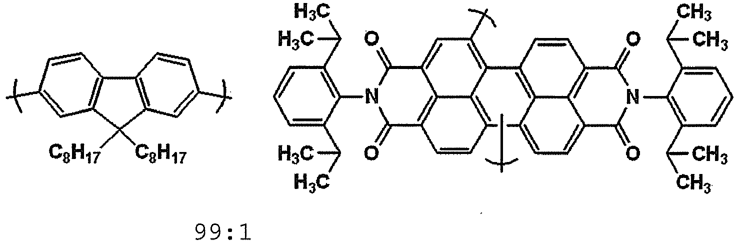

### <Synthesis of polymer compound 4>

Into a 200 mL separable flask were added 1.061 g (2.00 mmol) of 9,9-dioctylfluorene-2,7-diboric acid ethylene glycol ester, 0.987 g (1.80 mmol) of 9,9-dioctyl-2,7-dibromofluorene, 0.174 g (0.20 mmol) of N,N'-bis(2,6-diisopropylphenyl)-dibromoperylene-3,4:9,10-tetracarboxylic bisimide (an isomer mixture of a 1,6-dibromo body and a 1,7-dibromo body), 0.26 g of methyltrioctylammonium chloride (trade name: Aliquat 336, manufactured by Sigma Aldrich Japan) and 20 mL of toluene. Under a nitrogen atmosphere, 2.1 mg of bistriphenylphosphinepalladium dichloride was added and the mixture was heated at 85°C. This solution was heated at 105°C while dropping 5.4 mL of a 17.5 wt% sodium carbonate aqueous solution into this, then, the mixture was stirred for 6 hours. Next, 0.244 g of phenylboric acid and 20 mL of toluene were added, and the mixture was stirred at 105°C overnight.

After removal of the aqueous layer, 1.11 g of sodium N,N-diethyldithiocarbamate trihydrate and 22 mL ion exchanged water were added and the mixture was stirred at 85°C for 2 hours. The organic layer was separated from the aqueous layer, then, the organic layer was washed with ion exchanged water (twice), a 3 wt% acetic acid aqueous solution (twice) and ion exchanged water (twice) in this order.

The organic layer was dropped into methanol to cause precipitation of a polymer, and the precipitate was filtrated, then, dried to obtain a solid.

This solid was dissolved in toluene, and passed through a silica gel/alumina column through which toluene had been passed previously, the passed eluate was dropped into methanol to cause precipitation of a polymer, the precipitate was filtrated, then, dried to obtain 1.14 g of a polymer compound (hereinafter, referred to as polymer compound 4). The polystyrene-equivalent number-average molecular weight and weight average molecular weight were Mn=1.2×10⁴ and Mw=2.6×10⁴, respectively.

The polymer compound 4 is a polymer having the following repeating units at the following molar ratio (95:5)(the theoretical value calculated from raw materials).

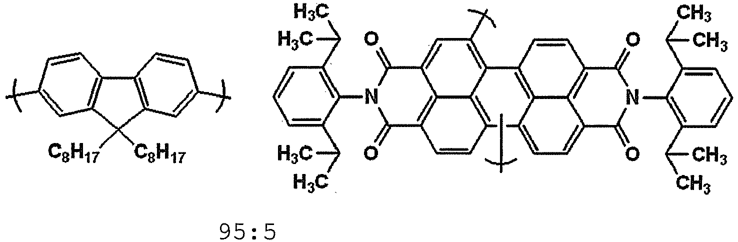

N,N'-bis(2,6-diisopropylphenyl)-dibromoperylene-3,4:9,10-tetracarboxylic bisimide (an isomer mixture of a 1,6-dibromo body and a 1,7-dibromo body) can be synthesized by a method described, for example, in Journal of Chemistry Vol. 70 (2005), p. 4323 to 4331.

### <Preparation of coating solution A1>

The polymer compound 1 was dissolved in xylene at a concentration of 1.0 wt%, thereafter, the solution was filtrated through a Teflon (registered trademark) filter having a pore size of 0.2µm, to prepare a coating solution A1.

### <Preparation of coating solution B>

The polymer compound 2 was dissolved in xylene at a concentration of 0.5 wt%, thereafter, the solution was filtrated through a Teflon (registered trademark) filter having a pore size of 0.2µm, to prepare a coating solution B.

### <Preparation of coating solution A2>

The polymer compound 1 was dissolved in xylene at a concentration of 1.0 wt%, further, the following compound BPPC (purchased from Sigma Aldrich Japan) was dissolved {polymer compound 1:compound BPPC = 100:0.1 (weight ratio)} as a perylene tetracarboxylic diimide derivative, to prepare a coating solution A2. 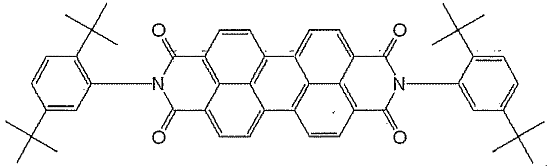 N,N'-Bis(2,5-di-tert-butylphenyl)-3,4,9,10-perylenedicarboximide
(abbreviation BPPC)

### <Preparation of coating solution A3>

The polymer compound 1 was dissolved in xylene at a concentration of 1.0 wt%, further, the above-described compound BPPC was dissolved {polymer compound 1:compound BPPC = 100:0.01 (weight ratio)}, to prepare a coating solution A3.

### <Preparation of coating solution A4>

The polymer compound 1 was dissolved in xylene at a concentration of 1.0 wt%, the polymer compound 3 was dissolved in chlorobenzene at a concentration of 1.0 wt%, further, these solutions were mixed so as to give a proportion of polymer compound 1:polymer compound 3 = 99:1 (weight ratio), to prepare a coating solution A4.

### <Preparation of coating solution A5>

The polymer compound 1 was dissolved in xylene at a concentration of 1.0 wt%, the polymer compound 3 was dissolved in chlorobenzene at a concentration of 1.0 wt%, further, these solutions were mixed so as to give a proportion of polymer compound 1:polymer compound 3 = 96:4 (weight ratio), to prepare a coating solution A5.

### <Preparation of coating solution A6>

The polymer compound 1 was dissolved in xylene at a concentration of 1.0 wt%, the polymer compound 4 was dissolved in chlorobenzene at a concentration of 1.0 wt%, further, these solutions were mixed so as to give a proportion of polymer compound 1:polymer compound 4 = 99:1 (weight ratio), to prepare a coating solution A6.

### (Fabrication organic EL device and evaluation thereof)

### <Example 1>

On a glass substrate carrying thereon an anode (ITO film having a thickness of 150 nm) formed on its surface by a sputtering method, a solution for forming a hole injection layer (manufactured by Plextronics, trade name: HIL764) was spin-coated, further, this was dried at 170°C for 15 minutes on a hot plate in atmospheric air, to form a hole injection (film thickness: 50 nm). Next, the coating solution B was spin-coated on the hole injection layer, and baked at 180°C for 60 minutes in a nitrogen atmosphere in a glove box, to form a hole transporting layer (film thickness: 20nm). Further, the above-described coating solution A2 was spin-coated on the hole transporting layer, to form a light emitting layer. Formation of a light emitting layer was regulated so that its thickness was 80 nm.

Thereafter, the layer was baked on a 130°C hot plate for 10 minutes in a nitrogen atmosphere, further, NaF was vapor-deposited with a thickness of 4 nm, then, Al was vapor-deposited with a thickness of 100 nm, there by forming a cathode.

The degree of vacuum in vapor deposition was in the range of 1×10⁻⁴ Pa to 9×10⁻³ Pa. The shape of the device was 2 mm×2 mm regular tetragon. The resultant device was driven under constant current at an initial luminance of 5000 cd/m², and a life test was carried out. The time until the luminance decreased to 4000 cd/m² (80% of the initial luminance) (this is called LT80) was measured. The measurement results are shown in Table 1.

### <Example 2>

An organic EL device was fabricated in the same manner as in Example 1 excepting that the coating solution A3 was used instead of the coating solution A2, and LT80 of the organic EL device was measured. The measurement results are shown in Table 1.

### <Example 3>

An organic EL device was fabricated in the same manner as in Example 1 excepting that the coating solution A4 was used instead of the coating solution A2, and LT80 of the organic EL device was measured. The measurement results are shown in Table 1.

### <Example 4>

An organic EL device was fabricated in the same manner as in Example 1 excepting that the coating solution A5 was used instead of the coating solution A2, and LT80 of the organic EL device was measured. The measurement results are shown in Table 1.

### <Example 5>

An organic EL device was fabricated in the same manner as in Example 1 excepting that the coating solution A6 was used instead of the coating solution A2, and LT80 of the organic EL device was measured. The measurement results are shown in Table 1.

### <Comparative Example 1>

An organic EL device was fabricated in the same manner as in Example 1 excepting that the coating solution A1 was used instead of the coating solution A2, and LT80 of the organic EL device was measured. The measurement results are shown in Table 1.

**Table 1**

| | Light emitting layer | LT80 (hrs) |
|---|---|---|
| Example 1 | Polymer compound 1/BPPC = 100/0.1 (weight ratio) | 5 |
| Example 2 | Polymer compound 1/BPPC = 100/0.01 (weight ratio) | 1 |
| Example 3 | Polymer compound 1/polymer compound 3 = 99:1 (weight ratio) | 2 |
| Example 4 | Polymer compound 1/polymer compound 3 = 96:4 (weight ratio) | 4 |
| Example 5 | Polymer compound 1/polymer compound 4 = 99:1 (weight ratio) | 5 |
| Comparative Example 1 | Polymer compound 1 | 0.6 |

As understood from Table 1, the organic EL device having a light emitting layer containing the first light emitting polymer (polymer compound 1) and perylene tetracarboxylic diimide derivative showed remarkable improvement in the LT80 life when driven under constant current at the same initial luminance, as compared with the organic EL device having a light emitting layer composed only of the first light emitting polymer. The organic EL device having a light emitting layer containing the second light emitting polymer (polymer compound 3 or polymer compound 4) showed remarkable improvement in the LT80 life when driven under constant current at the same initial luminance, as compared with the organic EL device having a light emitting layer composed only of the first light emitting polymer. That is, it was confirmed that the organic EL device having a light emitting layer containing the light emitting polymer composition of the present invention is excellent in device life.

### Industrial Applicability

According to the present invention, an organic EL device having improved device life can be realized.

## Claims

1. An organic electroluminescent device comprising a pair of electrodes and a light emitting layer disposed between the electrodes,
wherein the light emitting layer is
a light emitting layer comprising at least one compound selected from the group consisting of the following compounds (I) to (IV) and derivatives thereof, and a first light emitting polymer, or
a light emitting layer comprising a second light emitting polymer having as a repeating unit a divalent residue of at least one compound selected from the group consisting of the compounds (I) to (IV) and derivatives thereof:
(I) perylene tetracarboxylic diimide or naphthalene tetracarboxylic diimide
(II) perylene tetracarboxylic dianhydride or naphthalene tetracarboxylic dianhydride
(III) perylene tetracarboxylic bisimidazole or naphthalene tetracarboxylic bisimidazole
(IV) perylene tetracarboxylic bispyrimidine or naphthalene tetracarboxylic bispyrimidine.

2. The organic electroluminescent device according to Claim 1, wherein the derivatives of the compounds (I) to (IV) are polymer compounds.

3. The organic electroluminescent device according to Claim 1 or 2, wherein the first and second light emitting polymers are conjugated polymers.

4. The organic electroluminescent device according to any one of Claims 1 to 3, wherein the first and second light emitting polymers are light emitting polymers having as a repeating unit at least one diyl group selected from the group consisting of an unsubstituted or substituted fluorenediyl group and an unsubstituted or substituted benzofluorenediyl group.

5. The organic electroluminescent device according to any one of Claims 1 to 4, wherein the content of at least one compound selected from the group consisting of the compounds (I) to (IV) and derivatives thereof in the light emitting layer is 0.001 to 5 parts by weight when the content of the light emitting polymer is 100 parts by weight.

6. The organic electroluminescent device according to any one of Claims 1 to 5, wherein the content of at least one compound selected from the group consisting of the compounds (I) to (IV) and derivatives thereof in the light emitting layer is 0.01 to 1 part by weight when the content of the light emitting polymer is 100 parts by weight.

7. A light emitting apparatus comprising the organic electroluminescent device according to any one of Claims 1 to 6.

8. A light emitting polymer composition comprising at least one compound selected from the group consisting of the following compounds (I) to (IV) and derivatives thereof, and a first light emitting polymer, or comprising
a second light emitting polymer having as a repeating. unit a divalent residue of at least one compound selected from the group consisting of the compounds (I) to (YV) and derivatives thereof:
(I) perylene tetracarboxylic diimide or naphthalene tetracarboxylic diimide
(II) perylene tetracarboxylic diimide or naphthalene tetracarboxylic dianhydride
(III) perylene tetracarboxylic diimide or naphthalene tetracarboxylic bisimidazole
(IV) perylene tetracarboxylic bispyrimidine or naphthalene tetracarboxylic bispyrimidine.
